(19) **Europäisches Patentamt / European Patent Office / Office européen des brevets**

(11) **EP 4 575 466 A1**

(12) # EUROPEAN PATENT APPLICATION

(43) Date of publication:
**25.06.2025 Bulletin 2025/26**

(21) Application number: **23219351.6**

(22) Date of filing: **21.12.2023**

(51) International Patent Classification (IPC):
**G01N 21/64** (2006.01)

(52) Cooperative Patent Classification (CPC):
**G01N 21/6408; G01N 21/645; G01N 33/542;
G01N 33/54373; G01N 33/582;** G01N 21/6454;
G01N 21/648; G01N 2021/6432; G01N 2021/7786

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Stichting IMEC Nederland
5656 AE Eindhoven (NL)**

(72) Inventors:
• **VERSCHUEREN, Alwin Rogier Martijn
5221 LC 's-Hertogenbosch (NL)**
• **BESSELING, Thijs Herman
1181 TZ Amstelveen (NL)**
• **BEMBNOWICZ, Pawel
5581 SB Waalre (NL)**
• **NIKOLOVA, Dessislava
2150 Borsbeek (BE)**

(74) Representative: **AWA Sweden AB
Box 5117
200 71 Malmö (SE)**

(54) **A SENSOR DEVICE FOR SENSING A PRESENCE OF AN ANALYTE IN A BIOLOGICAL MATERIAL**

(57) A sensor device for sensing a presence of an analyte in a biological material, comprising: a plurality of sensor elements arranged at a sensing site, the plurality of sensor elements each comprising a photoluminescent probe and a bioreceptor configured to reversibly bind an analyte, wherein the binding of the analyte is configured to induce a conformational change of the sensor element, wherein the conformational change of the sensor element is configured to induce a change in photoluminescence lifetime of the photoluminescent probe; at least one photodetector being configured to sense the photoluminescence; a readout circuitry connected to the at least one photodetector and configured to readout a time resolved signal of photoluminescence sensed by the at least one photodetector, wherein the time resolved signal represents the photoluminescence lifetime of the photoluminescent probes of the plurality of sensor elements, and thereby represents presence of the analyte.

Fig. 1

## Description

Technical field

The present description relates to a sensor device for sensing a presence of an analyte in a biological material and a method for sensing a presence of an analyte in a biological material.

Background

In the prior art, in-vivo analyte biosensors are designed for long term monitoring and analysis of analytes in a biological material. The key requirements for the biosensors are selectivity, sensitivity, reversibility, biocompatibility and operational lifetime.

Several different biosensors are known. They may be categorized based on the bioreceptor used in the biosensor. The bioreceptor may be an enzyme, an antibody or an aptamer. Moreover, there are different ways to read-out a result from the biosensor. The read-out may be performed by using electrochemistry, photoluminescence, surface plasmon resonance, interferometry or field effect transistors.

Aptamers are short sequences of single-stranded DNA, RNA, synthetic XNA or peptide molecules that can be developed to interact with any desired target molecule with high affinity and specificity. An advantage of using aptamers is their selectivity, reversibility, stability, conformational change and generalizability for a very broad class of target analytes. However, using aptamers as bioreceptors in biosensors comes with a few limitations. The biosensor may suffer from signal drift, as the aptamers may detach from the surface in case of electrochemical read-out, or photobleaching of the photoluminescent probe in case of photoluminescence read-out, thereby decreasing the possible signal strength from the biosensor. Furthermore, aptamer-based biosensors have limited accuracy, due to the logarithmic dependence of the affinity binding on the target analyte concentration. Moreover, prior-art aptamer based in-vivo analyte biosensors based on a wire architecture [e.g. N. Arroyo-Curras et al., Real-time measurements of small molecules directly in awake ambulatory animals, PNAS, vol 114 (4), pp 645-650 (2017)] do not allow for straightforward extension to include multiple sensing sites for read-out of multiple aptamer variants in a single sensor device.

Thus, there is a need in the art for improvements.

Summary

An objective of the present description is to provide a sensor device for sensing a presence of an analyte in a biological material with a large operational lifetime of the sensor device. Moreover, an objective is to provide a method for sensing a presence of an analyte in a biological material.

According to an aspect, there is provided a sensor device for sensing a presence of an analyte in a biological material, said sensor device comprising:

a plurality of sensor elements arranged at a sensing site, the plurality of sensor elements each comprising a photoluminescent probe and a bioreceptor, the bioreceptor being configured to reversibly bind an analyte of the biological material, wherein the binding of the analyte of the biological material is configured to induce a conformational change of the sensor element, wherein the conformational change of the sensor element is configured to induce a change in photoluminescence lifetime of the photoluminescent probe;

at least one photodetector being configured to sense the photoluminescence of the photoluminescent probes;

a readout circuitry connected to the at least one photodetector and configured to readout a time resolved signal of photoluminescence sensed by the at least one photodetector, wherein the time resolved signal represents the photoluminescence lifetime of the photoluminescent probes of the plurality of sensor elements, and thereby represents presence of the analyte.

Thanks to the conformational change of the sensor elements and the change in photoluminescence induced by the change, the time resolved signal may indicate presence of the analyte. Thus, if no analyte is present, the photoluminescence lifetime of the photoluminescent probe may have a first characteristic value, whereas if an analyte is present, the photoluminescence lifetime of the photoluminescent probe may have a second characteristic value different from the first characteristic value. Hence, by determining the photoluminescence lifetime of photoluminescent probes, the sensor device may enable determining whether the analyte is bound to the sensor element or not.

The photoluminescence lifetime may be described as the average duration of the excited state of one photoluminescent probe until it decays by emitting a photon. In case the photoluminescent probe is excited using pulsed light, then the first and second characteristic values may for instance be derived from the time decay of the photoluminescence, such as a time period during which intensity of photoluminescence reduced to $1/e$ ($\approx 0.368$) in relation to an initial intensity after excitation of the photoluminescent probes.

However, it should be realized that other characteristic values may be used for representing photoluminescence lifetime. For instance, the photoluminescence decay may also be modelled by a stretched-exponential decay.

Thus, the time resolved signal may be read out in order to detect the presence of the analyte. The lifetime of the photoluminescence may provide a robust manner for determining presence of the analyte, with less signal drift compared to determining presence of an analyte by quantitatively measuring an intensity level of photoluminescence. In other words, even if the intensity level of the photoluminescent probe decreases over time, the dependence of the photoluminescence lifetime on the presence of the analyte is not changed and the time resolved signal may still provide necessary information for detecting the analyte. Hence, the sensor device based on photoluminescence lifetime measurement may have a long operational lifetime. The photoluminescence lifetime may be described as the average duration of the excited state of one photoluminescent probe until it decays by emitting a photon. The average duration is a material property of the specific photoluminescent probe, but it can be affected by the FRET (Förster Resonance Energy Transfer) mechanism, in which energy from the excited photoluminescent probe is transferred to a nearby (in nanometer range) molecule via dipole-dipole coupling. The binding of an analyte to a sensor element induces a conformational change of the sensor element. This conformational change may change the position of the photoluminescent probe in relation to the surrounding such that the photoluminescence lifetime of the photoluminescent probe is changed due to energy transfer from the photoluminescent probe to a molecule nearby. Different possible ways of this will be further described below.

The photoluminescence lifetime may also be described as a decay of an excited state of the photoluminescent probe, where the amount of emitted photoluminescence as function of time will be recorded by the photodetector. As the sensor device comprises a plurality of sensor elements, each with a photoluminescent probe and the possibility to bind an analyte, immediately after pulsed excitation, there is an initial high photoluminescence which decays after some time. The decay of the photoluminescence, or in other words the decay of the excited state of the photoluminescent probes, will depend on how many sensor elements have bound an analyte and thereby via their conformation exhibit a photoluminescence lifetime of first or second characteristic value. The total photoluminescence originating from the plurality of sensor elements arranged at a sensing site is detected by the photodetector.

The binding of the plurality of sensor elements to the analyte of the biological material happens when the bioreceptor of the sensor elements and the analyte of the biological material are in close proximity to each other, providing an interaction between the bioreceptor and the analyte of the biological material. The binding of the bioreceptor to the analyte of the biological material may for instance be a chemical or a physical binding of analytes by each bioreceptor of the plurality of sensor elements. The binding may thus include electrostatic interaction, hydrogen bonding, van der Waals forces or covalent binding.

The bioreceptors may comprise aptamers, antibodies or single domain antibodies.

The binding of the analyte to the sensor element induces a conformational change of the sensor element. There are several conformational changes possible as exemplified below. Further, the conformational change induces a change in the photoluminescence lifetime of the photoluminescent probe of the sensor element. The photoluminescence lifetime may be related to how energy transfer from the photoluminescent probe takes place, and the photoluminescence lifetime may be changed upon a conformational change of the sensor element.

The conformational change of the sensor element may move the photoluminescent probe closer to a substrate at which the sensor elements are immobilized. The photoluminescent probe may thereby transfer energy to the substrate, changing the lifetime of the photoluminescent probe. According to an alternative, energy may be transferred from the photoluminescent probe to the analyte being bonded to the sensor element and thus changing the photoluminescence lifetime. According to another alternative, energy may be transferred from the photoluminescent probe to a quencher, wherein the conformational change may cause the photoluminescent probe to be closer or farther away from the quencher, thus changing the photoluminescence lifetime. According to yet another alternative, energy may be transferred from the photoluminescent probe to a second photoluminescent probe, wherein the conformational change may cause the photoluminescent probe to be closer or farther away from the second photoluminescent probe, thus changing the photoluminescence lifetime.

The sensor element may be configured as a pair of sensor element parts, wherein one of the sensor element parts of the pair comprises a quencher. Upon binding of the analyte to the sensor element, there may be an energy transfer from the photoluminescent probe to the quencher, changing the lifetime of the photoluminescent probe.

The sensor element may be configured as a pair of sensor element parts, wherein each of the sensor element parts of the pair has a photoluminescent probe. Energy may be transferred from one photoluminescent probe to another photoluminescent probe, changing the lifetime of the photoluminescent probe.

The photoluminescent probe may have a lifetime before the conformational change of at least 20 nano seconds, however it should be understood that the photoluminescent probe may have a lifetime of up to seconds. The photoluminescent lifetime typically is longer if the photoluminescent probe is a phosphorescent probe compared to a fluorescent probe. The photoluminescent probe may have a lifetime after the conformational change of maximum 10 nanoseconds, such as 5 nanoseconds. The lifetime after the conformational change is of course highly depending on the lifetime before the change. A longer lifetime before the conformational change may give a longer lifetime after the conformational change, compared to a photoluminescent probe with a shorter lifetime as a starting value.

It should be realized that the sensor device may be used in several different applications and the sizing of the sensor device may be configured for the specific use.

The analyte may be ions, small molecules, such as nutrients or metabolites, and macromolecules, such as proteins or nucleic acids.

The biological sample may be an in-vivo or an in-vitro sample. In other words, the biological sample may be acquired from an in-vivo sample, such as a sample from a human or an animal. The sample may be acquired from the human or animal, or the sensor device may be mounted or arranged in the human or animal body. The biological sample may be acquired from an in-vitro sample, such as a cell culturing sample. Further, the sensor device may be mounted or arranged in the cell culturing chamber.

The photodetector may be any detector that is sensitive to light and able to record a signal in response to incident light with a high temporal resolution. For instance, the photodetector may comprise silicon or InGaAs PIN photodetector, i.e., a photodetector with an intrinsic region between a p-type and an n-type region. The photodetector may have a time resolution of nanoseconds, to be able to record the change in photoluminescence lifetime of the photoluminescent probe.

The at least one photodetector may be formed by an array of photodetectors configured to detect photoluminescence incident onto the array. Thus, a spatial resolution may be provided. In particular, if the sensor device includes a plurality of sensing sites, different photodetectors in the array may detect photoluminescence from different sensing sites in order to enable differentiating between photoluminescence from different sensing sites.

The photodetector may be configured to generate an electrical signal in response to sensing photoluminescence. The readout circuitry may be configured to read the electrical signal generated by the photodetector. The readout circuitry may thus be a suitable electrical circuitry and may for instance be formed by an integrated circuit. Such an integrated circuit may be an integrated circuit based on a printed circuit board or a complementary metal-oxide-semiconductor (CMOS) chip. The CMOS chip may come with an integrated signal processing (current to voltage conversion, amplification, bandpass filtering and phase detection).

Furthermore, the photodetector, the optical filters and readout circuit could all be monolithically integrated in a silicon CMOS chip.

The readout circuitry may be configured to process a signal from the photodetector in analog and/or digital domain.

The readout circuitry is connected to the at least one photodetector and configured to readout a time resolved signal of photoluminescence. The time resolved signal represents the photoluminescence lifetime of the photoluminescent probes of the plurality of sensor elements, and thereby represents presence of the analyte.

The time resolved signal may be used for determining whether there is any change in the photoluminescence lifetime of photoluminescent probes of the plurality of sensor elements. This may be used for determining presence of the analyte so as to determine the concentration of the analyte contained in the sample.

A larger concentration of the analyte in a sample implies that a larger fraction of the plurality of sensor elements bind the analyte. This implies that a larger fraction of the photoluminescent probes of the plurality of sensor elements will exhibit a second characteristic value of the photoluminescence lifetime. By measuring the time-dependent photoluminescence signal, this fraction between photoluminescent probes with first characteristic value and probes with second characteristic value for photoluminescence lifetime can be determined. From the fraction of photoluminescent probes with second compared to first characteristic value the analyte concentration can be determined.

According to one embodiment each of the plurality of sensor elements may comprise a bioreceptor of nucleotide or peptide type aptamer, wherein the aptamer may be configured to bind the analyte of the biological material.

Aptamers are short sequences of single-stranded DNA, RNA, synthetic XNA or peptide molecules that can be developed to interact with any desired target molecule with high affinity and specificity.

An advantage of using aptamers are their selectivity, reversibility, stability, conformational change and generalizability for a very broad class of target analytes.

It should be understood that a sensor element may be comprised of multiple sequences of aptamers connected to each other, in which at least one sequence is designed for binding the analyte, whereas the photoluminescent probe may be attached to another sequence designed to optimize the conformational change and the resulting change in the photoluminescence lifetime of the probe.

Alternatively, each of the plurality of sensor elements may comprise an antibody or single domain antibody wherein the photoluminescent probe may be attached to the antibody and wherein the antibody may be configured to bind the analyte of the biological material.

The photoluminescent probe may be attached to the bioreceptor of nucleotide or peptide type aptamer.

According to one embodiment the sensor device may comprise at least two groups of sensor elements, each group of sensor elements being arranged at a respective sensing site, wherein the groups of sensor elements have different bioreceptors.

In other words, a first group of sensor elements is arranged at a first sensing site and has a first type of bioreceptors, and a second group of sensor elements is arranged at a second sensing site and has a second type of bioreceptors. The first and second type of bioreceptors differ from each other.

For example, in case the bioreceptors are aptamers, the difference between the two types of aptamers may be their DNA sequence composition. Different type of bioreceptors may bind the same analyte or different analytes. By binding the same type of analyte, the accuracy, selectivity, sensitivity and operational concentration range of the sensor device towards the specific analyte is increased. By binding different analytes, more than one analysis may be performed at the same time.

There may be several groups of sensor elements with different bioreceptors, such as 10 or 100 groups of sensor elements. If so, multiple sensing sites with the respective sensor elements are configured on the sensor device.

According to one embodiment, the at least two groups of sensor elements may have a common type of photoluminescent probe.

By this, it is easy to analyze several groups of sensor elements with a common excitation light source and with an array of identical photodetectors, as the photoluminescence from all groups share the same wavelength characteristics. In addition, the photodetectors associated with different groups of sensor elements may be associated with a common filter for preventing excitation light to reach the photodetectors.

However, it should be understood that the at least two groups of sensor elements may have different kinds of photoluminescent probes. This may require different photodetectors for each group of sensor elements.

According to one embodiment, the at least two groups of sensor elements may be configured for having a high sensitivity and high accuracy for different concentrations of the same analyte of the biological material.

In other words, the at least two groups of sensor elements may be configured for having different equilibrium dissociation constants (kD) for the same analyte. Thus, the accuracy, selectivity, sensitivity and operational concentration range of the sensor device may be higher than having only one type of sensor elements.

According to one embodiment the sensor device may comprise at least two photodetectors, each photodetector may be configured to sense the photoluminescence from one of the at least two groups of sensor elements having different bioreceptors.

The different photodetectors may distinguish between signals from the different groups of sensor elements. Thus, having different groups of sensor elements binding different concentrations of analyte or binding different analytes may be distinguishable.

The at least two photodetectors may be arranged in an array of photodetectors. According to an alternative, the at least two photodetectors are separate from each other.

According to one embodiment the sensor device may further comprise a light source configured to output light illuminating the sensing site for exciting the photoluminescent probes.

The light source may be a laser light or a LED. The wavelength of the light source may match the excitation spectrum of the photoluminescent probe.

The sensor device may comprise several light sources, if photoluminescent probes of different kinds are used. For example, if there are at least two groups of sensor elements having different photoluminescent probes, then each group may have its own light source. Alternatively, the light source may be able to emit light of different wavelengths.

According to one embodiment the light source may be configured to output pulsed light or periodically modulated light.

The light source may be driven in a pulsed or periodic excitation mode.

In the pulsed mode, the light source is powered on for a short duration of time to bring the photoluminescent probe into its excited state. Then, using photodetectors, the decay of this excited state by the photoluminescent probes of the plurality of sensor elements, i.e., the amount of emitted photoluminescence as function of time, will be recorded.

In a pulsed mode, the light source may be configured to output a single pulse of excitation light. This may allow determining photoluminescence lifetime based on photoluminescence induced by a single short pulse.

In a periodic excitation mode, periodically modulated excitation light may be provided. This may imply that the photoluminescence output by the photoluminescent probes is also periodically modulated, which may facilitate determining the lifetime of the photoluminescent emission from the photoluminescent probes in a robust manner.

According to one embodiment the sensor device may further comprise a substrate having a first surface and a second surface, opposite to the first surface, wherein the sensing site may be arranged on the first surface of the substrate and the at least one photodetector may be arranged at the second surface of the substrate.

The substrate may comprise glass, preferably borosilicate glass. Alternatively, the substrate is comprised of quartz, silicon oxide, aluminum oxide, magnesium oxide, titanium oxide, zirconium oxide, silicon carbide, silicon nitride. Alternatively, the substrate may comprise any type of optically transparent material such as certain polymers, for example polycarbonate (PC), poly-methylmethacrylate (PMMA), or polyethylene terephthalate (PET).

The substrate may be transparent, at least partially for at least the excitation and emission wavelengths. The substrate and the positioning of the sensing site and the photodetector in relation to the substrate may be beneficial for spatial multiplexing of measurements at different sensing sites.

The at least one photodetector may be arranged on the second surface of the substrate, directly underneath the arrangement of the sensing site, being arranged on the first surface of the substrate. Thus, the at least one photodetector and the sensing site may be arranged in relation to each other.

According to one embodiment, the substrate may comprise a light guiding substrate and an optical filter, wherein the light guiding substrate may be configured for guiding excitation light to the sensing site by total internal reflection in the light guiding substrate.

The light guiding substrate may comprise an optically transparent material with refractive index larger than the fluid of the biological material. Typically, this means that the refractive index should be larger than n=1.33. The light guiding substrate may preferably comprise glass, such as borosilicate glass, having a refractive index of n=1.5.

Selecting the refractive index of the light guiding substrate to exceed the refractive index of the biological material allows for the excitation light originating from a light source located at the perimeter of the light guiding substrate to be distributed over the entire surface area of the light guiding substrate.

According to one embodiment, the sensing site may be arranged in a well of the light guiding substrate.

An advantage of this is that part of the excitation light may couple out of the light guiding substrate via vertical walls of the wells towards the sensor elements arranged at the sensing sites.

A further advantage is that the wells allow for a shorter distance between the sensor elements and the photodetectors, allowing for a smaller pitch between adjacent photodetectors without optical crosstalk.

According to one embodiment the plurality of sensor elements may be at least 1000 sensor elements per sensing site.

An advantage of this is that each sensing site comprises many sensor elements. By that, the total photoluminescent signal from each sensing site depends on the photoluminescence from a large number of photoluminescent probes. Thereby, there are more sensor elements that potentially can bind an analyte which improves the resolution with which the fraction can be determined of sensor elements that are bound to the analyte, and thereby improves the resolution with which the analyte concentration can be determined. Additionally, the signal strength is increased compared to having less sensor elements.

According to one embodiment the photoluminescent probe may comprise a fluorescent or phosphorescent dye, for example xanthene, rhodamine, fluorescein, naphthalimides, coumarins, cyanine, oxazines, pyrenes, acridines, porphyrins, metallo-porphyrins, lanthanide complexes, or quantum dots or nanoparticles comprising these compounds, being suitable photoluminescent probes.

According to one embodiment the sensor device may further comprise a processor device for processing the readout of the time resolved signal of photoluminescence sensed by the at least one photodetector from the readout circuitry, wherein the processor device may be configured to determine a fraction of sensor elements being subject to the conformational change based on the photoluminescence lifetime represented by the time resolved signal.

The fraction of sensor elements being subject to the conformational change may be converted to a concentration of the analyte.

According to one embodiment the sensor device further comprises a carrier, the carrier may be arranged for carrying the sensor element, the photodetector and the read-out circuitry, the carrier may be configured for being implantable or ingestible.

Thus, the sensor device on the carrier may be configured for being ingested by a human or an animal. By this, analytes of the intestinal tract of the user can be analyzed. Further, the sensor device on the carrier may be implanted such that it can analyze for instance blood flow of the user. The sensor device on the carrier may be configured for being a transdermal patch or an arterial catheter.

The sensor device on the carrier may alternatively be configured for use in fluid environments outside a living being, such as in water, water waste pipes or in bioreactors.

The sensor device on the carrier may alternatively be configured for use as a fluid sensor connected to extracorporeal tubing, a fluid sensor connected to a cell culturing chamber, a bioreactor or a microphysiological system.

The sensor device on the carrier may be mounted or arranged in the cell culturing chamber.

In general the sensor device on the carrier may be used as in-line sensor, at-line sensor or probe for monitoring concentrations of chemical and biochemical compounds in any liquid.

This includes monitoring of drinking water, environmental water, industrial process water, beverage production, etcetera.

According to another aspect, there is provided a method for sensing a presence of an analyte in a biological material, said method comprising:

capturing an analyte of the biological material by a plurality of sensor elements arranged at a sensing site, the plurality of sensor elements each comprising a photoluminescent probe and a bioreceptor, the bioreceptor being configured to reversibly bind the analyte, wherein the binding of the analyte of the biological material is configured to induce a conformational change of the sensor element, wherein the conformational change of the sensor element is configured to induce a change in photoluminescence lifetime of the photoluminescent probe;

sensing, by at least one photodetector, the photoluminescence of the photoluminescent probes;

reading, by a readout circuitry connected to the at least one photodetector, a time resolved signal of photoluminescence sensed by the at least one photodetector, wherein the time resolved signal represents the photoluminescence lifetime of the photoluminescent probes of the plurality of sensor elements, and thereby represents presence of the analyte.

This aspect may generally present the same or corresponding advantages as the former aspect.

The method allows for a sensing of the presence of an analyte in a biological material, wherein sensing is not sensitive to signal drift, has high accuracy and may be able to use for repeated measurements.

The capturing of the analyte by the plurality of sensor element happens when the bioreceptor of the sensor elements and the analyte of the biological material are in close proximity to each other, providing an interaction between the bioreceptor and the analyte of the biological material. The binding of the bioreceptor to the analyte of the biological material may for instance be a chemical or a physical binding of analytes by each bioreceptor of the plurality of sensor elements. The binding may thus include electrostatic interaction, hydrogen bonding, van der Waals forces or covalent binding.

The capturing of the analyte to the sensor element induces a conformational change of the sensor element. There are several conformational changes possible as exemplified below. Further, the conformational change induces a change in the photoluminescence lifetime of the photoluminescent probe of the sensor element. The photoluminescence lifetime may be related to how energy transfer from the photoluminescent probe takes place, and the photoluminescence lifetime may be changed upon a conformational change of the sensor element.

The conformational change of the sensor element may move the photoluminescent probe closer to a substrate at which the sensor elements are immobilized. The photoluminescent probe may thereby transfer energy to the substrate, changing the lifetime of the photoluminescent probe. According to an alternative, energy may be transferred from the photoluminescent probe to the analyte being bonded to the sensor element and thus changing the photoluminescence lifetime. According to another alternative, energy may be transferred from the photoluminescent probe to a quencher, wherein the conformational change may cause the photoluminescent probe to be closer or farther away from the quencher, thus changing the photoluminescence lifetime. According to yet another alternative, energy may be transferred from the photoluminescent probe to a second photoluminescent probe, wherein the conformational change may cause the photoluminescent probe to be closer or farther away from the second photoluminescent probe, thus changing the photoluminescence lifetime.

The sensor element may be configured as a pair of sensor element parts, wherein one of the sensor element parts of the pair contains a quencher. Upon binding of the analyte to the sensor element, there may be an energy transfer from the photoluminescent probe to the quencher, changing the lifetime of the photoluminescent probe.

The sensor element may be configured as a pair of sensor element parts, wherein each of the sensor element parts of the pair has a photoluminescent probe. Energy may be transferred from one photoluminescent probe to another photoluminescent probe, changing the lifetime of the photoluminescent probe.

Thus, the conformational change induced by the capturing the analyte induces a change in photoluminescence lifetime of the photoluminescent probe.

The at least one photodetector senses the photoluminescence of the photoluminescent probes.

The photodetector may be configured to generate an electrical signal in response to sensing photoluminescence. The readout circuitry may be configured to read the electrical signal generated by the photodetector. The readout circuitry may thus be a suitable electrical circuitry and may for instance be formed by an integrated circuit. The readout circuitry may be configured to process a signal from the photodetector in analog and/or digital domain.

Thus, the read out circuitry reads the time resolved signal. The time resolved signal represents the photoluminescence lifetime of the photoluminescent probes and thereby the presence of analytes.

Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.

Fig. 1 is a cross section of a sensor device according to the present invention.
Fig. 2a-d discloses operation of the sensor device using a periodically modulated excitation light source.
Fig 2e discloses operation of the sensor device using a pulsed excitation light source.
Fig. 3a and b are cross sections of a sensor device according to the present invention.

Fig. 4a-g are cross sections of a sensor device according to the present invention.

Fig. 5a and b are cross sections of a sensor device according to the present invention.

Fig. 6 is a schematic view of an ingestible device according to the present invention.

Fig. 7 is a schematical view of a method according to the present invention.

Detailed description

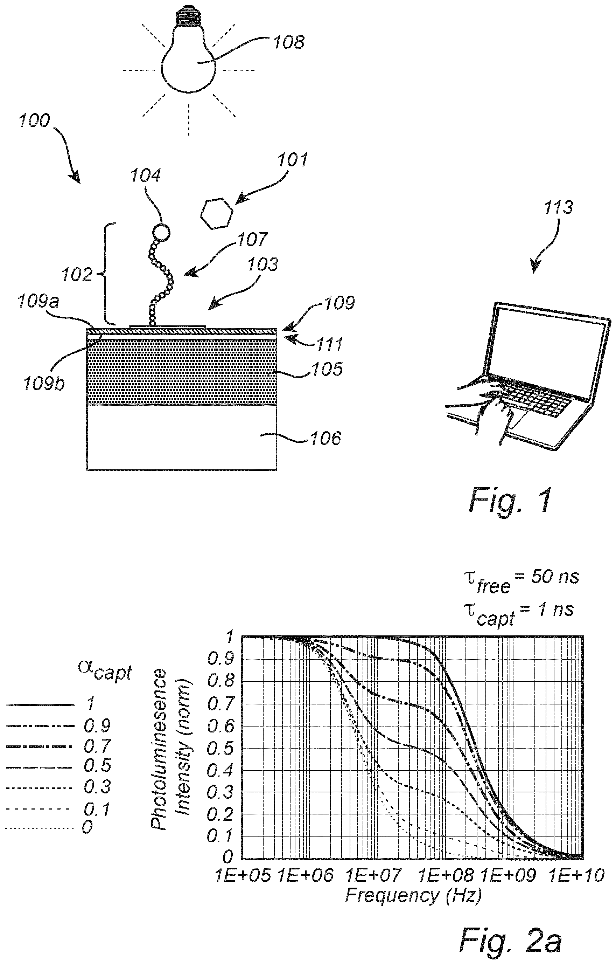

Fig. 1 illustrates a cross section of a sensor device 100 for sensing a presence of an analyte 101 in a biological material. The analyte 101 may be ions, small molecules, such as nutrients or metabolites, and macromolecules, such as proteins or nucleic acids.

The sensor device 100 comprises a plurality of sensor elements 102. The plurality of sensor elements 102 may be at least 1000 sensor elements 102. The sensor elements 102 are arranged at a sensing site 103. The sensing site 103 is an area of a substrate 109, where the sensor elements 102 are immobilized. The sensing site 103 may be coated with biotin groups. The sensor elements 102 may bind to these biotin groups using avidin or streptavidin protein groups contained in the sensor elements 102. Alternatively, the sensing site 103 may be azide functionalized, to which alkyne groups contained in the sensor elements 102 can bind.

The plurality of sensor elements 102 each comprises a photoluminescent probe 104.

The sensor device 100 may further comprise a substrate 109 having a first surface 109a and a second surface 109b, the second surface being opposite to the first surface 109a. The sensing site 103 is arranged on the first surface 109a of the substrate 109 and the at least one photodetector 105 is arranged at the second surface 109b of the substrate 109.

The substrate 109 may comprise glass, preferably borosilicate glass. Alternatively, the substrate is comprised of quartz, silicon oxide, aluminum oxide, magnesium oxide, titanium oxide, zirconium oxide, silicon carbide, silicon nitride, Alternatively, the substrate may comprise any type of optically transparent material such as certain polymers, for example polycarbonate (PC), poly-methylmethacrylate (PMMA), or polyethylene terephthalate (PET).

The sensor device 100 may comprise an optical filter 111. The optical filter 111 may be chosen such that it filters out wavelengths which are not originating from the photoluminescent probe 104. In other words, the optical filter 111 is chosen such that it only passes photoluminescent light from the photoluminescent probes 104. This optical filter 111 can be based on absorption, but preferably is a dichroic or interference filter, that reflects (and not absorbs) all light outside the wavelength range matched to the photoluminescent probe 104. Such filters comprise multiple thin layers of dielectric materials having alternating high and low refractive indices.

The photoluminescent probe 104 comprises a fluorescent or phosphorescent dye, for example xanthene, rhodamine, fluorescein, naphthalimides, coumarins, cyanine, oxazines, pyrenes, acridines, porphyrins, metallo-porphyrins or lanthanide complexes. The photoluminescent probe 104 may comprise quantum dots or nanoparticles comprising the above-mentioned compounds.

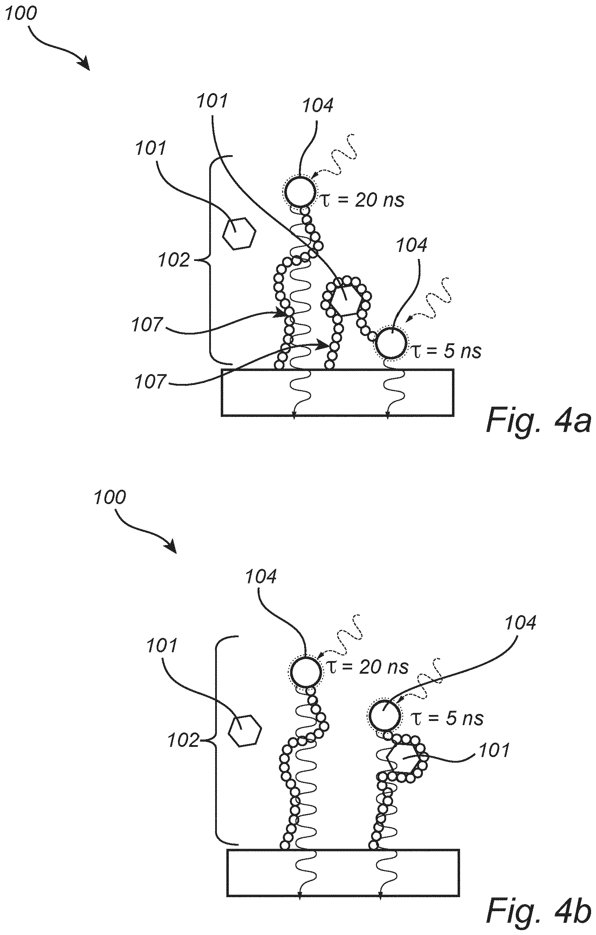

The plurality of sensor elements 102 are configured to reversibly bind an analyte 101 of the biological material. The reversibility of the binding of the analyte 101 to the bioreceptor 107 comprised in the sensor elements 102 allows for the sensor device 100 to be continuously used or to be re-used. The binding of the analyte 101 of the biological material is configured to induce a conformational change of the sensor element 102. The conformational change of the sensor element 102 is configured to induce a change in photoluminescence lifetime of the photoluminescent probe 104. The conformational change is discussed in more detail in relation to Figs 4a-g.

The photoluminescence lifetime may be described as the average duration of the excited state of one photoluminescent probe 104 until it decays by emitting a photon. The average duration is a material property of the specific photoluminescent probe, but it can be affected by the FRET (Förster Resonance Energy Transfer) mechanism, in which energy from the excited photoluminescent probe is transferred to a nearby (in nanometer range) molecule via dipole-dipole coupling.

The photoluminescence lifetime may also be described as a decay of an excited state of the photoluminescent probe 104, where the amount of emitted photoluminescence as function of time will be recorded by the photodetector 105. As the sensor device 100 comprises a plurality of sensor elements 102, each with a photoluminescent probe 104 and the possibility to bind an analyte 101, immediately after pulsed excitation, there is an initial high photoluminescence which decays after some time. The decay of the photoluminescence, or in other words the decay of the excited state of the photoluminescent probes 104, will depend on how many sensor elements 102 have bound an analyte 101 and thereby via their conformation exhibit a photoluminescence lifetime of first or second characteristic value. The total photoluminescence originating from the plurality of sensor elements arranged at a sensing site is detected by the photodetector 105.

For example, the photoluminescent probe in its unbound conformation may have a photoluminescence lifetime of at least 20 nano seconds. The photoluminescent probe may have a reduced photoluminescence lifetime after the conformational change due to binding an analyte of for example maximum 10 nanoseconds, such as 5 nanoseconds.

The plurality of sensor elements 102 may comprise a bioreceptor 107, preferably an aptamer. The photoluminescent probe 104 may be attached to the bioreceptor 107 and the bioreceptor 107 may be configured to bind the analyte 101 of the biological material. Aptamers are short sequences of single-stranded DNA, RNA, synthetic XNA or peptide molecules that can be developed to interact with any desired target molecule with high affinity and specificity.

The sensor device 100 further comprises at least one photodetector 105 being configured to sense the photoluminescence of the photoluminescent probes 104.

The sensor device 100 further comprises a readout circuitry 106. The readout circuitry 106 is connected to the at least one photodetector 105 and configured to readout a time resolved signal of photoluminescence sensed by the at least one photodetector 105.

The time resolved signal represents the photoluminescence lifetime of the photoluminescent probes 104 of the plurality of sensor elements 102, and thereby represents presence of the analyte 101.

The sensor device 100 may further comprise a light source 108 configured to output light illuminating the sensing site 103 for exciting the photoluminescent probes 104. The light source 108 may be a laser light or a LED. The wavelength of the light source 108 may match the excitation spectrum of the photoluminescent probe. The light source 108 may be configured to output pulsed light or periodically modulated light.

In the pulsed mode, the light source 108 is powered for a short duration. The duration may for instance be shorter than the photoluminescence lifetime of the photoluminescent probe 104. When the light source 108 is powered for the short duration, the photoluminescent probes 104 are excited and following the excited state is the decay of the excited state, which is recorded by the photodetector 105.

The periodic modulated excitation mode is described in relation to Fig. 2a-d. The light source 108 is modulated periodically such that it is modulated with for instance a sinewave of set excitation frequency. By this, the photoluminescent output may further also become periodically modulated. The photodetector 105 will then record of the photoluminescent light emitted from the photoluminescent probe 104 both the intensity and the phase relative to the excitation signal. From the recorded intensity and phase, the photoluminescence lifetime may be obtained using Equation 1.

$$I_{exc} = I_0 \cdot e^{jwt} \rightarrow V_{emit} = I_0 \cdot e^{jwt} \cdot \left[ \frac{1 - \alpha_{capt}}{1 + j\omega\tau_{free}} + \frac{\alpha_{capt}}{1 + j\omega\tau_{capt}} \right] \qquad (1)$$

Where $I_{exc}$ is the intensity of the modulated excitation signal (without the constant baseline), $\alpha_{capt}$ is the fraction of sensor elements 102 having bound an analyte 101, $\tau_{free}$ is the photoluminescence lifetime of the photoluminescent probe 104 before capturing an analyte 101, $\tau_{capt}$ is the photoluminescence lifetime of the photoluminescent probe 104 after capturing an analyte 101, j is the unit imaginary number, $\omega$ is $2\pi$ times the excitation frequency, e is Eulers number used as exponential base, $I_0$ is the maximum of the intensity of the excitation signal, $V_{emit}$ is the scaled modulated photoluminescence emission (without constant baseline) combined from the fractions of bound and unbound sensing elements 102.

Fig 2a, illustrates the excitation frequency response of the recorded photoluminescent intensity for sensor devices 100 with different $\alpha_{capt}$, i.e where the sensor devices 100 have different fractions of sensor elements 102 having bound an analyte 101. For example, the photoluminescence lifetime of the photoluminescent probe 104 before the sensor element 102 captures an analyte 101, $\tau_{free}$ is 50 ns, while the photoluminescence lifetime of the photoluminescent probe 104 after binding of an analyte 101, $\tau_{capt}$ is 1 ns. For example, for the intermediate case $\alpha_{capt} = 0.5$, where half of the sensor elements 102 are in the unbound free state, and the other half has captured and bound an analyte 101, there are clearly two characteristic frequencies observable in which the photoluminescence intensity makes a sharp intensity reduction. These characteristic frequencies correspond to the inverse of the photoluminescent lifetimes $\tau_{free}$ and $\tau_{capt}$ multiplied by factor $2\pi$.

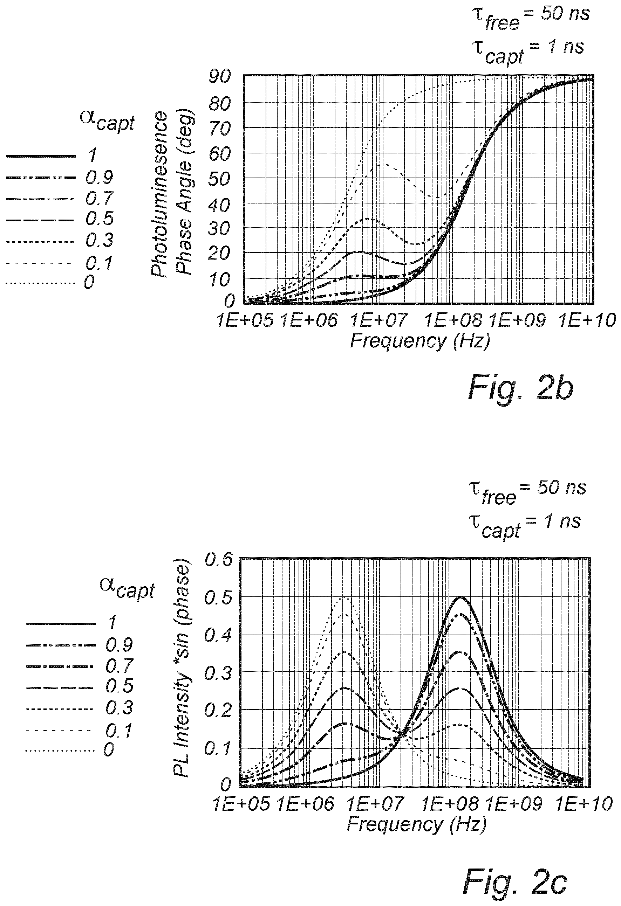

Fig 2b, illustrates the excitation frequency response of the recorded photoluminescence phase angle for sensor devices 100 with different $\alpha_{capt}$, i.e where the sensor devices 100 have different fractions of sensor elements 102 having bound an analyte 101. The photoluminescence lifetime of the photoluminescent probe 104 before the sensor element 102 captures an analyte 101, $\tau_{free}$ is 50 ns, while the photoluminescence lifetime of the photoluminescent probe 104 after binding of an analyte 101, $\tau_{capt}$ is 1 ns. Again, two characteristic frequencies may be observed when the analyte 101 is captured by the sensor element 102.

Fig 2c, illustrates the excitation frequency response of the combined recorded photoluminescence intensity and photoluminescence phase angle (by using the intensity multiplied by the sin(phase angle)) for sensor devices 100 with different $\alpha_{capt}$, i.e where the sensor devices 100 have different fractions of sensor elements 102 having bound an analyte 101. The photoluminescence lifetime of the photoluminescent probe 104 before the sensor element 102 captures an analyte 101, $\tau_{free}$ is 50 ns, while the photoluminescence lifetime of the photoluminescent probe 104 after binding of an analyte 101, $\tau_{capt}$ is 1 ns. Again, the two characteristic frequencies are even more emphasized.

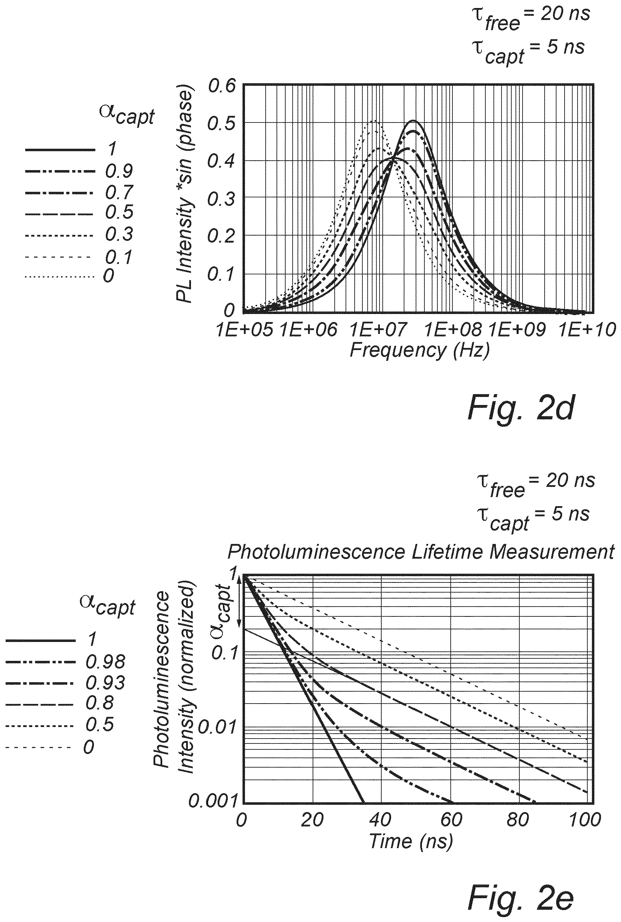

Fig 2d, illustrates the excitation frequency response of the combined recorded photoluminescence intensity and photoluminescent phase angle (by using the intensity multiplied by the sin(phase angle)) for sensor devices 100 with different $\alpha_{capt}$, i.e where the sensor devices 100 have different fractions of sensor elements 102 having bound an analyte 101. The photoluminescence lifetime of the photoluminescent probe 104 before the sensor element 102 captures an

analyte 101, $\tau_{free}$ is 20 ns, while the photoluminescence lifetime of the photoluminescent probe 104 after binding of an analyte 101, $\tau_{capt}$, is 5 ns. Thus, even with a closer $\tau_{free}$ and $\tau_{capt}$ the two characteristic frequencies can be observed, showing the robustness of the device 100. This figure 2d illustrates how experimentally, using the above combination of photoluminescence intensity and phase, by comparing its measured value at the first characteristic frequency (at the inverse of the photoluminescent lifetime $\tau_{free}$ multiplied by factor $2\pi$) to its value at the second characteristic frequency (at the inverse of the photoluminescent lifetime $\tau_{capt}$ multiplied by factor $2\pi$), the fraction $\alpha_{capt}$ of sensor elements 102 having bound an analyte 101 can be determined.

The pulsed excitation mode is described in relation to Fig. 2e. The light source 108 emits a pulse of excitation light. Immediately after pulsed excitation, there is an initial high photoluminescence which decays after some time. The photodetector 105 will then record the total decay of the photoluminescence emitted from the photoluminescent probe 104. The photoluminescence intensity $I_{PL}$ in the pulsed mode is described in Equation 2.

$$I_{PL} = \left(1 - \alpha_{capt}\right) \cdot e^{-\frac{t}{\tau_{free}}} + \alpha_{capt} \cdot e^{-\frac{t}{\tau_{capt}}} \quad (2)$$

Where $I_{PL}$ is the photoluminescence intensity, $\alpha_{capt}$ is the fraction of sensor elements 102 having bound an analyte 101, $\tau_{free}$ is the photoluminescence lifetime of the photoluminescent probe 104 before capturing an analyte 101, $\tau_{capt}$ is the photoluminescence lifetime of the photoluminescent probe 104 after capturing an analyte 101.

In Fig. 2e, $\tau_{free}$ is 20 ns and $\tau_{capt}$ is 5 ns. For example, for the intermediate case $\alpha_{capt}$ = 0.8, where 20% of the sensor elements 102 are in the unbound free state, and the remaining 80% have captured and bound an analyte 101, the decay curve of the total photoluminescence intensity in Fig. 2e clearly shows two characteristic decay periods: an initial fast decay with characteristic time constant $\tau_{capt}$, followed by a slow decay with characteristic time constant $\tau_{free}$. The relative contributions of these 2 periods are determined by $\alpha_{capt}$. As can be seen in Fig. 2e, with a larger fraction $\alpha_{capt}$ of the sensor elements 102 having bound to an analyte 101, the initial fast decay becomes more pronounced than compared to the case of no analytes 101 bound. This figure 2e illustrates how experimentally from the measured decay of the photoluminescence intensity after pulsed excitation, by extrapolating the measured slow decay to the initial moment at which the fast decay has started, the fraction $\alpha_{capt}$ of sensor elements 102 having bound an analyte 101 can be determined.

Returning again to Fig. 1, the sensor device 100 may further comprise a processor device 113 for processing the readout of the time resolved signal of photoluminescence sensed by the at least one photodetector 105 from the readout circuitry 106. The processor device 113 is configured to determine a fraction of sensor elements 102 being subject to the conformational change based on the photoluminescence lifetime represented by the time resolved signal.

The processor device 113 may be a central processing unit (CPU), which may execute the instructions of one or more computer programs in order to provide desired functionality.

The processor device 113 may alternatively be implemented as firmware arranged e.g. in an embedded system, or as a specifically designed processing unit, such as an Application-Specific Integrated Circuit (ASIC) or a Field-Programmable Gate Array (FPGA), which may be configured to implement desired functionality.

The processor device 113 may be arranged in a common housing with the at least one photodetector 105 and the readout circuitry 106 and may be configured to receive the time resolved signal directly from the readout circuitry 106 for processing the time resolved signal. However, according to an alternative, the time resolved signal may be communicated through wired or wireless communication to the processor device 113. Thus, the processor device 113 may be physically arranged anywhere in relation to the at least one photodetector 105 and the readout circuitry 106, such as being arranged in a server "in the cloud".

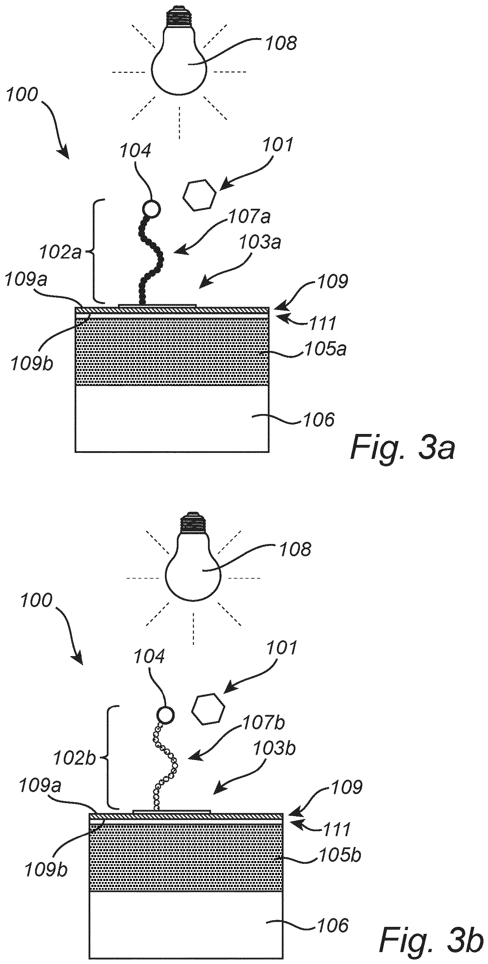

Fig. 3 illustrates the sensor device 100 comprising at least two groups of sensor elements 102a, 102b. For simplicity, features already presented in connection to Fig. 1 will not be repeated.

It should be understood that the number of groups may be larger than two. It may for instance be up to 10 or 100 number of groups. Each group of sensor elements 102a, 102b are arranged at a respective sensing site 103a, 103b. The groups of sensor elements 102a, 102b have different bioreceptors 107a, 107b, preferably aptamers.

As can be seen in Fig. 3 the first group of sensor elements 102a has a first type of bioreceptors 107a, and the second type of sensor elements 102b has a second type of bioreceptors 107b.

The first 107a and second type 107b of bioreceptors differ from each other. For example, in case of aptamer bioreceptors, the difference between the two types of aptamers 107a, 107b may be their DNA sequence composition. Different type of bioreceptors 107a, 107b may bind the same analyte 101 or different analytes.

The at least two groups of sensor elements 102a, 102b may be configured for having a high sensitivity and high accuracy for different concentrations of the same analyte 101 of the biological material. In other words, the at least two groups of sensor elements 102a, 102b may be configured for having different equilibrium dissociation constants (kD) for the same analyte 101.

The dissociation constant may be described as follows. kD=kOFF/kON. At every moment in time there is chance that a nearby analyte 101 binds to the bioreceptor 107, expressed by an association rate constant kON. There is also a chance that the bound analyte 101 is released, expressed by a dissociation rate constant kOFF. In equilibrium the association and dissociation rates become equal, which determines the fraction of sensor elements 102 with bound analytes 101 depending on the analyte concentration. Stated differently at an analyte 101 concentration equal to kD(=kOFF/kON) the fraction of bound sensor elements 102 is 50%.

The at least two groups of sensor elements 102a, 102b may have a common type of photoluminescent probe 104.

However, it should be understood that the at least two groups of sensor elements 102a, 102b may have different kinds of photoluminescent probes 104. This may require different photodetectors 105a, 105b and different optical filters 111 for each group of sensor elements 102a, 102b. However, using different kinds of photoluminescent probes 104, the groups of sensor elements 102a, 102b may emit photoluminescence of different wavelengths, which may facilitate avoiding crosstalk in measuring photoluminescence from the different groups of sensor elements 102a, 102b.

The sensor device 100 may then also comprise several light sources 108. For example, if there are at least two groups of sensor elements 102a, 102b having different photoluminescent probes 104, then each group may have its own light source 108. Alternatively, the light source 108 may be able to emit light of different wavelengths.

The sensor device 100 may further comprise at least two photodetectors 105a, 105b. Each photodetector 105a, 105b may then be configured to sense the photoluminescence from one of the at least two groups of sensor elements 102a, 102b having different bioreceptors 107a, 107b. The different photodetectors 105a, 105b may distinguish between signals from the different groups of sensor elements 102a, 102b.

The at least two photodetectors 105a, 105b may be arranged in an array of photodetectors 105a, 105b.

Fig. 4a-g illustrate different sensor elements 102 being configured to make different conformational changes when binding an analyte 101. The figures should be interpreted as being the same sensor element 102 before and after binding of an analyte 101.

The binding of the analyte 101 to the sensor element 102 induces a conformational change of the sensor element 102. There are several conformational changes possible as exemplified below. Further, the conformational change induces a change in the photoluminescence lifetime of the photoluminescent probe 104 of the sensor element 102. The photoluminescence lifetime may be related to how the energy transfer from the photoluminescent probe 104 takes place, and the photoluminescence lifetime may be changed upon a conformational change of the sensor element 102.

Fig. 4a discloses how the conformational change of the sensor element 102 moves the photoluminescent probe 104 closer to substrate 109 and thereby transferring energy to the substrate 109, changing the lifetime of the photoluminescent probe 104 from a first photoluminescence lifetime when no analyte 101 is bound to a second photoluminescence lifetime when an analyte 101 is bound to the sensor element 102.

Fig. 4b discloses how energy may be transferred from the photoluminescent probe 104 to the analyte 101 which is bound to the sensor element 102 and by that changes the photoluminescence lifetime of the photoluminescent probe 104 from a first photoluminescence lifetime when no analyte 101 is bound to a second photoluminescence lifetime when an analyte 101 is bound to the sensor element 102.

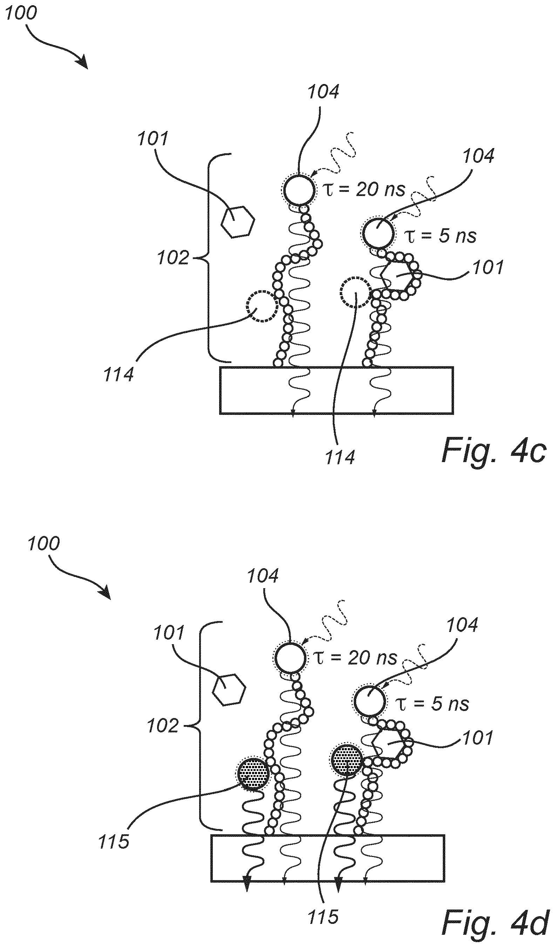

Fig. 4c discloses how energy is transferred from the photoluminescent probe 104 to a quencher 114. The quencher 114 may be attached to the sensor element 102. The quencher 114 may be a molecule being able to absorb energy from the photoluminescent probe 104 and re-emit the energy as heat or light. The quencher 114 may comprise dimethylaminoazosulphonic acid (Dabcyl) or Black Hole Quenchers™. The conformational change may cause the photoluminescent probe 104 to move from a farther distance from the quencher 114 into being closer to the quencher 114 allowing energy transfer from the photoluminescent probe 104 to the quencher 114 after the conformational change. Thus, this changes the photoluminescence lifetime of the photoluminescent probe 104 from a first photoluminescence lifetime when no analyte 101 is bound to a second photoluminescence lifetime when an analyte 101 is bound to the sensor element 102.

Fig. 4d discloses how energy is transferred from the photoluminescent probe 104 to a second photoluminescent probe 115. The second photoluminescent probe 115 may be attached to the sensor element 102. When the analyte 101 is bound and the sensor element 102 changes the conformation, the photoluminescent probe 104 is closer to the second photoluminescent probe 115 and energy may be transferred from the photoluminescent probe 104 to the second photoluminescent probe 115. Thus, this changes the photoluminescence lifetime of the photoluminescent probe 104 from a first photoluminescence lifetime when no analyte 101 is bound to a second photoluminescence lifetime when an analyte 101 is bound to the sensor element 102.

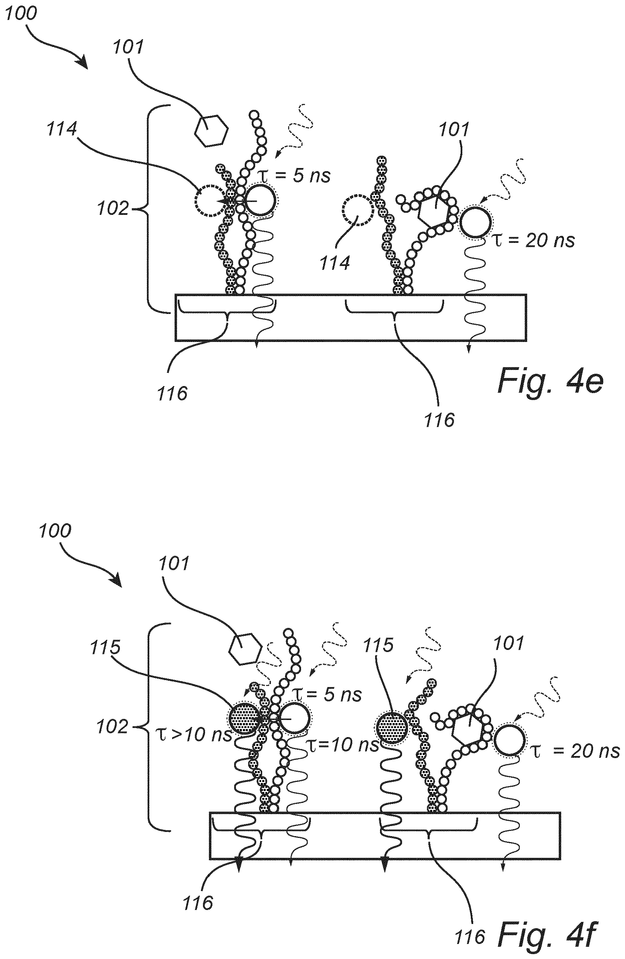

Fig. 4e discloses how the sensor element 102 may be configured as a pair of sensor element parts 116, wherein one of the sensor element parts of the pair comprises a quencher 114. The pair of sensor element parts 116 may be two aptamers. The quencher 114 may be a molecule being able to absorb energy from the photoluminescent probe 104 and re-emit the energy as heat or light. The quencher 114 may comprise Dabcyl or Black Hole Quenchers™. The conformational change may cause the photoluminescent probe 104 to move from a closer distance from the quencher 114 into being farther to the quencher 114 reducing the energy transfer from the photoluminescent probe 104 to the quencher 114 after the conformational change. This changes the lifetime of the photoluminescent probe 104 from a first photoluminescence lifetime when no analyte 101 is bound to a second photoluminescence lifetime when an analyte 101 is bound to the sensor element 102.

Fig. 4f discloses how the sensor element 102 may be configured as a pair of sensor element parts 116, wherein one of the sensor element parts of the pair comprises a photoluminescent probe 104 and the other comprises a second photoluminescent probe 115. Before the analyte 101 is bound to one of the sensor elements 102 of the sensor element parts 116, part of the energy of excited photoluminescent probe 104 is transferred to the second photoluminescent probe 115, giving the photoluminescent probe 104 a first photoluminescence lifetime. However, after the binding of the analyte 101 to the sensor element 102, the conformational change moves the photoluminescent probe 104 farther away from the second photoluminescent probe 115, reducing the energy transfer to the second photoluminescent probe 115, and giving the photoluminescent probe 104 a second photoluminescence lifetime.

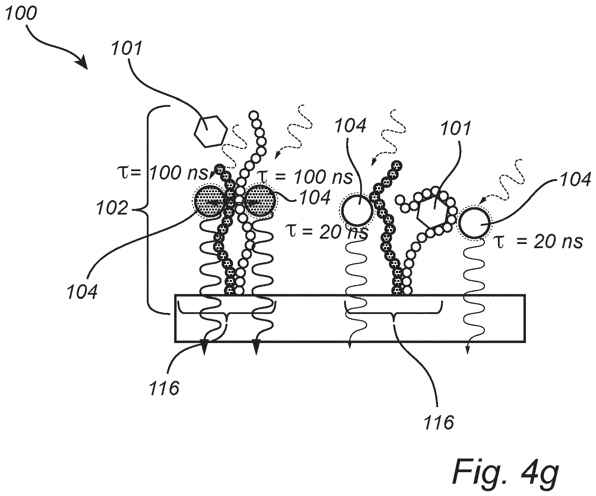

Fig 4g. discloses how the sensor element 102 may be configured as a pair of sensor element parts 116, wherein both of the sensor element parts of the pair comprise an identical photoluminescent probe 104. Before the analyte 101 is bound to one of the sensor elements 101, the two photoluminescent probes 104 being in close proximity to each other interact as a dimer and have a first photoluminescence lifetime. After the analyte 101 is bound, the conformational change of the sensor element 102 disrupts the interaction and the photoluminescent probes 104 have a second photoluminescence lifetime.

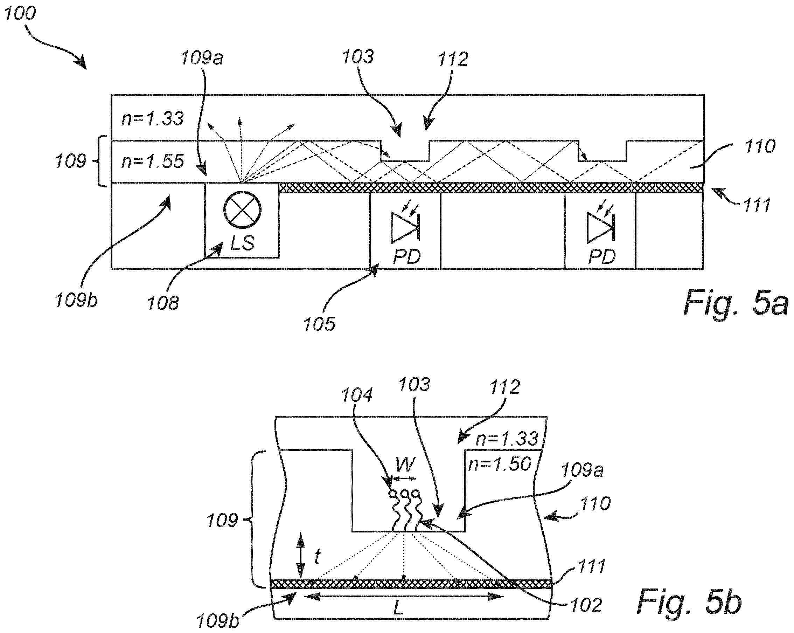

Fig. 5a and 5b illustrate how the substrate 109 may further be comprised of a light guiding substrate 110 and an optical filter 111. The light guiding substrate 110 is configured for guiding excitation light to the sensing site 103 by total internal reflection in the light guiding substrate 110. The sensing site 103 may be arranged in a well 112 of the light guiding substrate 110.

The well 112 is more closely depicted in Fig 5b. By having the sensing site 103 arranged in the well 112, the photoluminescent probes 104 being close to the surface of the well 112 may be effectively excited, reducing the relative contribution of background photoluminescence that may result from other material in the biological sample liquid. Moreover, the photoluminescent probes 104 within the well 112 are also arranged closer to the photodetector 105, such that the width L to which the emitted photoluminescence is spread onto the photodetector 105 may become more narrow.

This is an advantage if the sensor device 100 comprises several photodetectors 105, since each photodetector 105 may have its own well 112 separated laterally with a pitch distance at least equal to the width L in order to avoid crosstalk between the photodetectors 105. Thus, smaller thickness t below the well, allows for a smaller pitch distance L, and therefore for a larger number of sensing sites 103 and photodetectors 105 per sensor surface area. This improves the selectivity, sensitivity and accuracy of the sensor device 100.

The width, w, of sensing site 103 covered with sensor elements 102 inside the well 112 may be in the range of 1 to 10 micrometer

The thickness of the light guiding substrate below the well, t, may be in the range of 10 to 100 micrometer. Then, the separation between neighboring wells and photodetectors is given by a pitch distance L of 50 to 500 micrometer. And with that the well size should be in between w and L, typically in the range of 100 to 300 micrometer.

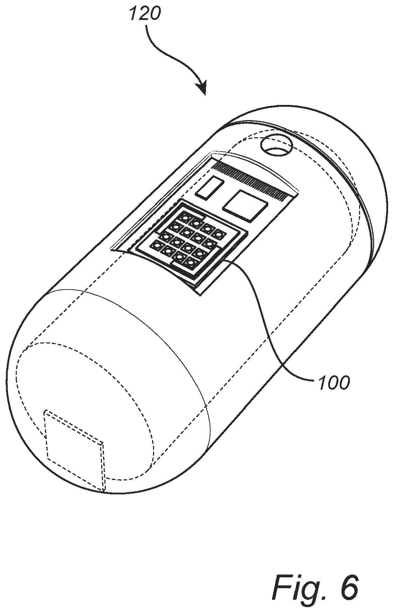

Fig. 6 discloses a sensor device 100 according to above, further comprising a carrier 120. The carrier 120 being arranged for carrying the sensor element 102, the photodetector 105 and the read-out circuitry 106. The sensor device 100 may further comprise a processor device 113. However, a processor device 113 may alternatively be arranged anywhere in relation to the at least one photodetector 105 and the readout circuitry 106, such as being arranged in a server "in the cloud". It may communicate with the photodetector 105 and the read out circuitry 106 through wired or wireless communication.

The carrier 120 is configured for being implantable or ingestible, meaning that it is possible to arrange and package the components of the sensor device 100 such that it may be able to analyze analytes 101 inside a body of a human or an animal. The sensor device 100 on the carrier 120 may be configured for being a transdermal patch or an arterial catheter.

The carrier 120 may alternatively be configured for use in fluid environments outside a living being, such as in water, water waste pipes or in bioreactors. Thus, it is possible to arrange and package the components of the sensor device 100 such that it may be able to analyze analytes 101 in water, water waste pipes or in bioreactors.

The sensor device 100 on the carrier 120 may alternatively be configured for use as a fluid sensor connected to extracorporeal tubing, a fluid sensor connected to a cell culturing chamber, a bioreactor or a microphysiological system.

The sensor device 100 on the carrier 120 may be mounted or arranged in the cell culturing chamber.

The sensor device 100 may then benefit from having a long operational lifetime, being possible thanks to the photoluminescence lifetime measurements. This allows for use of the sensor device 100 for a long time after the ingestion or implanting of the sensor device 100.

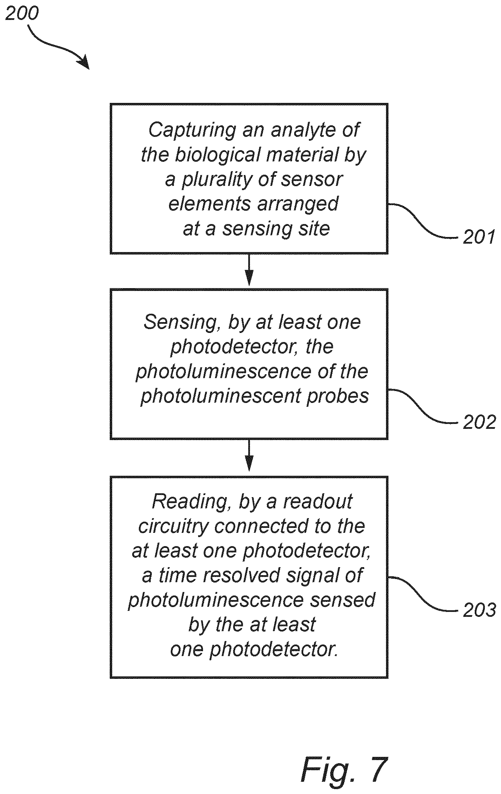

Fig. 7 discloses a method 200 for sensing a presence of an analyte 101 in a biological material.

The method 200 comprises capturing 201 an analyte 101 of the biological material by a plurality of sensor elements 102 arranged at a sensing site 103.

The plurality of sensor elements 102 each comprises a photoluminescent probe 104 and bioreceptor 107. The analyte 101 is captured by bioreceptor 107 comprised in the plurality of sensor elements 102 being configured to bind the analyte 101. It should be understood that the analyte 101 is captured by a fraction of the total amount of sensor elements 102. How large fraction of sensor elements has bound an analyte 101 depends on the concentration of the analyte 101 and the affinity of the analyte 101 to the sensor element 102. The binding of the analyte 101 of the biological material is configured to induce a conformational change of the sensor element 102. The conformational change of the sensor element 102 is configured to induce a change in photoluminescence lifetime of the photoluminescent probe 104.

The method further comprises sensing 202, by at least one photodetector 105, the photoluminescence of the photoluminescent probes 104.

The method further comprises reading 203, by a readout circuitry 106 connected to the at least one photodetector 105, a time resolved signal of photoluminescence sensed by the at least one photodetector 105. The time resolved signal represents the photoluminescence lifetime of the photoluminescent probes 104 of the plurality of sensor elements 102, and thereby represents presence of the analyte 101.

The method thus includes reading a plurality of sensor elements 102 each comprising a photoluminescent probe 104. When the analyte 101 is present, for a fraction of sensor elements 102 the photoluminescence lifetime changes depending on how many sensor elements have captured the analyte 101. Thus, a detection of the change of the total time resolved photoluminescence signal originating from the plurality of the photoluminescent probes 104 tells if the analyte 101 is present.

Moreover, for the case of pulsed excitation, the method analyses how the intensity of the photoluminescence from the plurality of the photoluminescent probes 104 decays over time. Depending on the total time resolved decay, it can be determined what fraction of sensor elements 102 have bound an analyte 101. This may in turn be translated into a concentration of the analyte 101, by using the probability of the analyte 101 to bind to a sensor element 102.

Alternatively, for the case of periodically modulated excitation, the method analyses how the intensity and phase of the photoluminescence from the plurality of the photoluminescent probes 104 varies with the excitation frequency. From the excitation frequency dependence, it can be determined what fraction of sensor elements 102 have bound an analyte 101. This may in turn be translated into a concentration of the analyte 101, by using the probability of the analyte 101 to bind to a sensor element 102.

In the above the inventive concept has mainly been described with reference to a limited number of examples. However, as is readily appreciated by a person skilled in the art, other examples than the ones disclosed above are equally possible within the scope of the inventive concept, as defined by the appended claims.

**Claims**

1.  A sensor device (100) for sensing a presence of an analyte (101) in a biological material, said sensor device (100) comprising:

    a plurality of sensor elements (102) arranged at a sensing site (103), the plurality of sensor elements (102) each comprising a photoluminescent probe (104) and a bioreceptor (107), the bioreceptor (107) being configured to reversibly bind an analyte (101) of the biological material, wherein the binding of the analyte (101) of the biological material is configured to induce a conformational change of the sensor element (102), wherein the conformational change of the sensor element (102) is configured to induce a change in photoluminescence lifetime of the photoluminescent probe (104);
    at least one photodetector (105) being configured to sense the photoluminescence of the photoluminescent probes (104);
    a readout circuitry (106) connected to the at least one photodetector (105) and configured to readout a time resolved signal of photoluminescence sensed by the at least one photodetector (105), wherein the time resolved signal represents the photoluminescence lifetime of the photoluminescent probes (104) of the plurality of sensor elements (102), and thereby represents presence of the analyte (101).

2.  A sensor device (100) according to claim 1, wherein each of the plurality of sensor elements (102) comprises a bioreceptor (107) of oligonucleotide or peptide type aptamer wherein the aptamer (107) is configured to bind the analyte (101) of the biological material.

3.  A sensor device (100) according to claim 1 or 2, wherein the sensor device (100) comprises at least two groups of sensor elements (102a, 102b), each group of sensor elements (102a, 102b) being arranged at a respective sensing site (103a, 103b), wherein the groups of sensor elements (102a, 102b) have different bioreceptors (107a, 107b).

4. A sensor device (100) according to claim 3, wherein the at least two groups of sensor elements (102a, 102b) have a common type of photoluminescent probe (104).

5. A sensor device (100) according to any one of claims 3 or 4, wherein the at least two groups of sensor elements (102a, 102b) are configured for having a high sensitivity and high accuracy for different concentrations of the same analyte (101) of the biological material.

6. A sensor device (100) according to any one of claims 3 to 5, wherein the sensor device (100) comprises at least two photodetectors (105a, 105b), each photodetector (105a, 105b) being configured to sense the photoluminescence from one of the at least two groups of sensor elements (102a, 102b) having different bioreceptors (107a, 107b).

7. A sensor device (100) according to any one of claims 1 to 6, wherein the sensor device (100) further comprises a light source (108) configured to output light illuminating the sensing site (103) for exciting the photoluminescent probes (104).

8. A sensor device (100) according to claim 8, wherein the light source (108) is configured to output pulsed light or periodically modulated light.

9. A sensor device (100) according to any one of claims 1 to 8, wherein the sensor device (100) further comprises a substrate (109) having a first surface (109a) and a second surface (109b), opposite to the first surface (109a), wherein the sensing site (103) is arranged on the first surface (109a) of the substrate and the at least one photodetector (105) is arranged at the second surface (109b) of the substrate.

10. A sensor device (100) according to claim 9, wherein the substrate (109) comprises a light guiding substrate (110) and an optical filter (111), wherein the light guiding substrate (110) is configured for guiding excitation light to the sensing site (103) by total internal reflection in the light guiding substrate (110), and wherein the sensing site (103) is arranged in a well (112) of the light guiding substrate (110).

11. A sensor device (100) according to any one of claims 1 to 10, wherein the plurality of sensor elements (102) are at least 1000 sensor elements (102) per sensing site (103).

12. A sensor device (100) according to any one of claims 1 to 11, wherein the photoluminescent probe (104) comprises a fluorescent or phosphorescent dye, for example xanthene, rhodamine, fluorescein, naphthalimides, coumarins, cyanine, oxazines, pyrenes, acridines, porphyrins, metallo-porphyrins, lanthanide complexes, or quantum dots or nanoparticles comprising these compounds.

13. A sensor device (100) according to any one of claims 1 to 12, wherein the sensor device (100) further comprises a processor device (113) for processing the readout of the time resolved signal of photoluminescence sensed by the at least one photodetector (105) from the readout circuitry (106), wherein the processor device (113) is configured to determine a fraction of sensor elements (102) being subject to the conformational change based on the photoluminescence lifetime represented by the time resolved signal.

14. A sensor device (100) according to any one of claims 1 to 13, further comprising a carrier (120), the carrier (120) being arranged for carrying the sensor elements (102), the photodetector (105) and the read-out circuitry (106), the carrier (120) being configured for being used as an intracorporeal implant, an ingestible pill, a transdermal patch, an arterial catheter, a fluid sensor connected to extracorporeal tubing, a fluid sensor connected to a cell culturing chamber, a bioreactor or a microphysiological system.

15. A method (200) for sensing a presence of an analyte (101) in a biological material, said method (200) comprising:

capturing (201) an analyte (101) of the biological material by a plurality of sensor elements (102) arranged at a sensing site (103), the plurality of sensor elements (102) each comprising a photoluminescent probe (104), and a bioreceptor (107), the bioreceptor (107) being configured to reversibly bind the analyte (101), wherein the binding of the analyte (101) of the biological material is configured to induce a conformational change of the sensor element (102), wherein the conformational change of the sensor element (102) is configured to induce a change in photoluminescence lifetime of the photoluminescent probe (104);
sensing (202), by at least one photodetector (105), the photoluminescence of the photoluminescent probes (104);
reading (203), by a readout circuitry (106) connected to the at least one photodetector (105), a time resolved signal of photoluminescence sensed by the at least one photodetector (105), wherein the time resolved signal represents the photoluminescence lifetime of the photoluminescent probes (104) of the plurality of sensor elements (102), and thereby represents presence of the analyte (101).

Fig. 1

Fig. 2a

Fig. 2b

Fig. 2c

$\tau_{free} = 20\ ns$
$\tau_{capt} = 5\ ns$

$\alpha_{capt}$

| | |
|---|---|
| —————— | 1 |
| —·—··— | 0.9 |
| —·—·— | 0.7 |
| — — — | 0.5 |
| ·········· | 0.3 |
| - - - - | 0.1 |
| ············ | 0 |

Fig. 2d

$\tau_{free} = 20\ ns$
$\tau_{capt} = 5\ ns$

Photoluminescence Lifetime Measurement

$\alpha_{capt}$

| | |
|---|---|
| —————— | 1 |
| —·—··— | 0.98 |
| —·—·— | 0.93 |
| — — — | 0.8 |
| ·········· | 0.5 |
| - - - - | 0 |

Fig. 2e

18

Fig. 3a

Fig. 3b

100

101
101
104
104

102

107

$\tau = 20 \ ns$

$\tau = 5 \ ns$

107

*Fig. 4a*

100

104
104

101

102

101

$\tau = 20 \ ns$

$\tau = 5 \ ns$

*Fig. 4b*

Fig. 4c

Fig. 4d

*Fig. 4e*

*Fig. 4f*

Fig. 4g

Fig. 5a

Fig. 5b

EP 4 575 466 A1

Fig. 6

*200*

Capturing an analyte of
the biological material by
a plurality of sensor
elements arranged
at a sensing site

*201*

Sensing, by at least one
photodetector, the
photoluminescence of the
photoluminescent probes

*202*

Reading, by a readout
circuitry connected to the
at least one photodetector,
a time resolved signal of
photoluminescence sensed
by the at least
one photodetector.

*203*

*Fig. 7*

Europäisches
Patentamt
European
Patent Office
Office européen
des brevets

# EUROPEAN SEARCH REPORT

**Application Number**

EP 23 21 9351

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/333101 A1 (HEIKENFELD JASON CHARLES [US]) 19 October 2023 (2023-10-19) | 1,2,7, 11,12, 14,15 | INV. G01N21/64 |
| Y | * paragraphs [0010], [0034] - [0041] * <br> * figures 2,4 * | 6,9,10 | |
| X | US 2016/278638 A1 (SCHWARTZ JERROD JOSEPH [US] ET AL) 29 September 2016 (2016-09-29) | 1-5,7,8, 12,15 | |
| Y | * paragraphs [0072], [0083], [0100], [0106], [0147] * <br> * figures 3B,6C * | 6,9,10 | |
| X | US 2019/353663 A9 (UNIV MINNESOTA [US]) 21 November 2019 (2019-11-21) <br> * paragraphs [0013], [0021] - [0023], [0034], [0091], [0107], [0108], [0114] - [0117], [0255] * <br> * figures 6A,6B,8B,19D * | 1,7,8, 11-13,15 | |
| Y | WO 2023/173001 A1 (EGLINT INC [US]; ARMANIOUS MIENA [US] ET AL.) 14 September 2023 (2023-09-14) <br> * paragraphs [0040], [0044], [0253], [0254] * <br> * claim 27 * <br> * figures 12A-C * | 6,9 | **TECHNICAL FIELDS SEARCHED (IPC)** <br><br> G01N |
| Y | US 2021/121874 A1 (ROTHBERG JONATHAN M [US] ET AL) 29 April 2021 (2021-04-29) <br> * paragraphs [0303], [0305], [0310] * <br> * figures 6D,6F * | 9,10 | |

The present search report has been drawn up for all claims

| Place of search | Date of completion of the search | Examiner |
|---|---|---|
| Munich | 31 May 2024 | D'Alessandro, Davide |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 23 21 9351

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2024

| Patent document cited in search report | | Publication date | Patent family member(s) | | Publication date |
|---|---|---|---|---|---|
| US 2023333101 | A1 | 19-10-2023 | NONE | | |
| US 2016278638 | A1 | 29-09-2016 | US | 2016278638 A1 | 29-09-2016 |
| | | | WO | 2016154034 A1 | 29-09-2016 |
| US 2019353663 | A9 | 21-11-2019 | US | 2018238901 A1 | 23-08-2018 |
| | | | US | 2021255195 A1 | 19-08-2021 |
| WO 2023173001 | A1 | 14-09-2023 | NONE | | |
| US 2021121874 | A1 | 29-04-2021 | AU | 2020372908 A1 | 02-06-2022 |
| | | | BR | 112022008098 A2 | 12-07-2022 |
| | | | CA | 3159566 A1 | 06-05-2021 |
| | | | CN | 114930028 A | 19-08-2022 |
| | | | EP | 4051903 A1 | 07-09-2022 |
| | | | JP | 2023502329 A | 24-01-2023 |
| | | | KR | 20220101108 A | 19-07-2022 |
| | | | US | 2021121874 A1 | 29-04-2021 |
| | | | WO | 2021086985 A1 | 06-05-2021 |

For more details about this annex : see Official Journal of the European Patent Office, No. 12/82

**REFERENCES CITED IN THE DESCRIPTION**

**Non-patent literature cited in the description**

- **N. ARROYO-CURRAS et al.** Real-time measurements of small molecules directly in awake ambulatory animals. *PNAS*, 2017, vol. 114 (4), 645-650